(19) Europäisches Patentamt
European Patent Office
Office européen des brevets

(11) **EP 2 201 937 A1**

(12) **EUROPEAN PATENT APPLICATION**

(43) Date of publication:
**30.06.2010 Bulletin 2010/26**

(51) Int Cl.:
*A61K 9/20* (2006.01)   *A61K 9/50* (2006.01)
*A61K 31/59* (2006.01)   *A61K 31/663* (2006.01)

(21) Application number: **09180698.4**

(22) Date of filing: **23.12.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **23.12.2008 US 140345 P**

(71) Applicant: **TEVA PHARMACEUTICAL INDUSTRIES LTD.**
**49131 Petah Tiqva (IL)**

(72) Inventors:
• **Shakib, Leon**
  **Holon (IL)**
• **Fox, Michael**
  **67291, Tel-Aviv (IL)**

(74) Representative: **Russell, Tim**
  **D Young & Co LLP**
  **120 Holborn**
  **London EC1N 2DY (GB)**

(54) **Formulations comprising vitamin D or derivatives thereof**

(57) The present invention provides stable formulations of vitamin D or a derivative thereof, preferably cholecalciferol.

**Description**

Field of the Invention

The invention relates to formulations of vitamin D or derivatives thereof, preferably cholecalciferol, and processes for preparing the same. In particular, the present invention provides stable formulations of vitamin D or derivatives thereof, preferably cholecalciferol, and processes for preparing the same.

Background of the Invention

Cholecalciferol is a form of vitamin D, also referred to as vitamin D3. Reportedly, cholecalciferol is used in the correction of calcium and vitamin D deficiency in the elderly. Also, cholecalciferol may be used as an adjunct to specific therapy for osteoporosis, in patients with either established vitamin D and calcium combined deficiencies or in those patients at high risk of needing such therapeutic supplements.

For example, alendronate/cholecalciferol combinations are marketed in the UK as Fosavance®, apparently, for use in the treatment of postmenopausal osteoporosis in patients at risk of vitamin D deficiency.

Alendronate is a active ingredient that is part of a group of drugs referred to as bisphosphonates.

During treatment with bisphosphonates, the early inhibition of bone resorption, apparently, induces a decrease in serum calcium, which occurs within days to weeks of the start of treatment. The serum calcium decrease can persist for many weeks to months following the initiation of treatment and can be prominent in vitamin D-insufficient patients. The hypocalcemic response can occasionally be severe enough to be symptomatic and warrant clinical intervention, particularly in patients with hypoparathyroidism and in cancer patients (see Vasikaran, S.D., Ed., 30 Bisphosphonates: An Overview with Special Reference to Alendronate, Ann. Clin.-4, Biochem. (2001)' 38: 608-623). As a result, adequate vitamin D (e.g. cholecalciferol) and calcium intake is recommended for subjects using bisphosphonates. Vitamin D supplementation becomes even more critical when calcium needs are elevated due to the net influx of calcium into bone that occurs as a result of bisphosphonate therapy during effective osteoporosis treatment. Reportedly, adequate vitamin D intake is essential to facilitate intestinal absorption of calcium, plays a critical role in regulating calcium metabolism, and is critically important in the mineralization of the skeleton. The primary biological function of vitamin D is to maintain calcium homeostasis by increasing the intestine's efficiency in absorbing dietary calcium and thereby helping ensure that the amount of calcium absorbed is adequate to maintain blood calcium in the normal range and adequate to maintain skeletal mineralization.

However, cholecalciferol is seen to be very unstable and is especially unstable in the presence of oxygen. It has therefore been difficult to provide formulations of cholecalciferol which are stable. This lack of stability may often be detected as a drop in the level of cholecalciferol in a formulation measured using a cholecalciferol assay.

Cholecalciferol, formulations containing cholecalciferol, and process for their preparation have been known since the 1950's.

More recently, WO03/059358 describes oil compositions containing an oil and 25-hydroxy vitamin D3 in which the pharmaceutical active ingredient is dissolved in an oil.

US 4,997,824 describes soft gelatine capsules containing cholecalciferol derivatives in combination with other active ingredients.

It would therefore be highly desirable to provide stable pharmaceutical formulations comprising cholecalciferol. In particular, it would be highly desirable to provide stable solid pharmaceutical formulations comprising cholecalciferol.

Brief Description of the Drawings

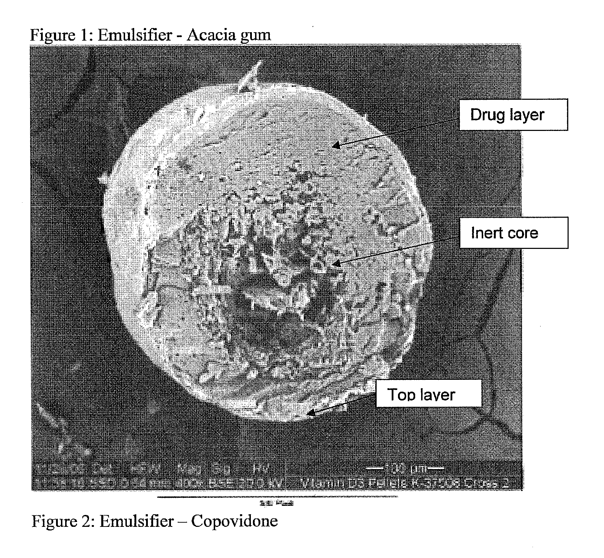

Figure 1 illustrates a cross section of the pharmaceutical delivery system in accordance with the present invention when acacia gum was used as an emulsifier;

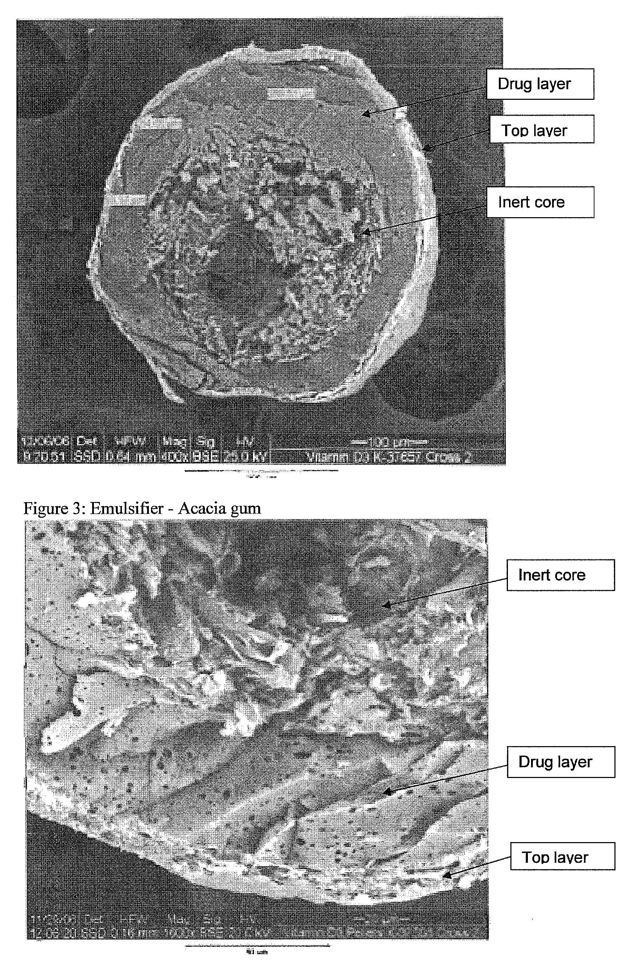

Figure 2 illustrates a cross section of the pharmaceutical delivery system in accordance with the present invention when copovidone was used as an emulsifier;
Figure 3 provide a closer view of a cross section of the pharmaceutical delivery system in accordance with the present invention when acacia gum was used as an emulsifier;

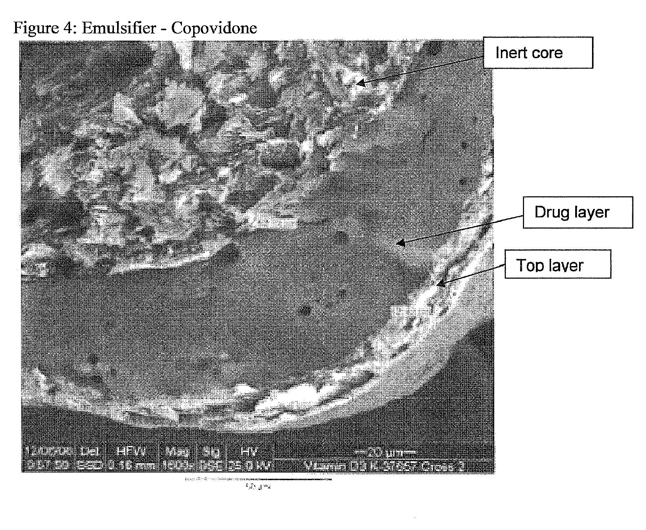

Figure 4 provide a closer view of a cross section of the pharmaceutical delivery system in accordance with the present invention when copovidone was used as an emulsifier;

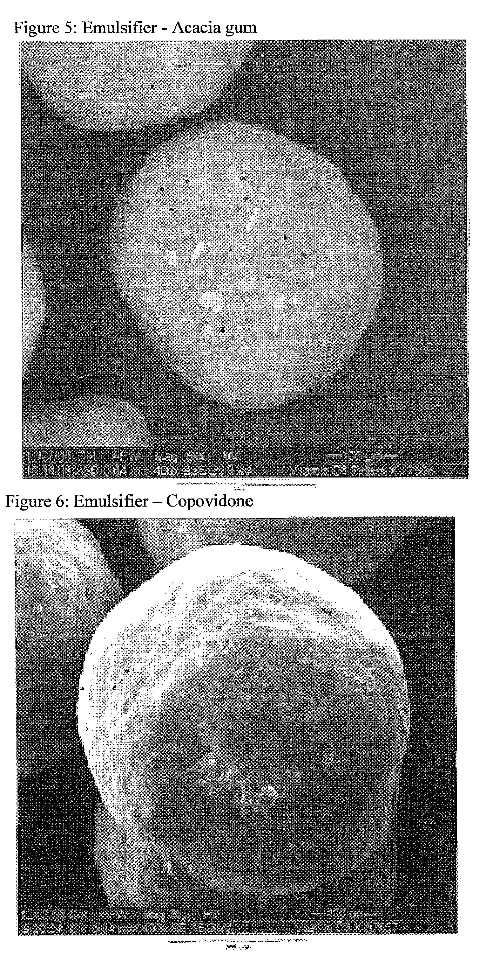

Figure 5 illustrates an outside view of the pharmaceutical delivery system in accordance with the present invention when acacia gum was used as an emulsifier;
Figure 6 illustrates an outside view of the pharmaceutical delivery system in accordance with the present invention when copovidone was used as an emulsifier;

Summary of the Invention

The present invention provides a stable pharmaceutical delivery system comprising vitamin D or derivatives thereof, preferably cholecalciferol.

In a first aspect, the present invention provides a pharmaceutical delivery system comprising:

i) an inert core,
ii) an inner layer comprising vitamin D or a derivative thereof, preferably cholecalciferol, an emulsifier and an antioxidant, and
iii) an outer protective layer.

In a second aspect, the present invention provides a pharmaceutical composition comprising a pharmaceutical delivery system according to any embodiment of the first aspect of the present invention described herein.

In particular, the pharmaceutical composition further comprises a second active pharmaceutical ingredient. Preferably, the pharmaceutical composition of the present invention further comprises a bisphosphonate such as alendronate, risedronate, ibandronate, zolendronate or a salt thereof.

Detailed Description of the Invention

The present invention provides a stable pharmaceutical grade delivery system comprising vitamin D or derivatives thereof, preferably cholecalciferol.

As used herein "pharmaceutical grade" means produced using validated and well-controled production procedure.

As used herein "medium chain triglycerides " are medium-chain (6 to 12 carbons) fatty acid esters of glycerol.

As used herein "vitamin D" means vitamin D, isomers or derivatives thereof, or combinations of vitamin D and its derivatives. Preferably the vitamin D derivative is cholecalciferol.

As used herein, the term "bisphosphonate" describes a group of active pharmaceutical ingredients. The meaning of the term is well-known to the person skilled in the art. Preferred bisphosphonates include alendronate, risedronate, ibandronate, zolendronate or salts thereof. A preferred bisphosphonate is alendronate or salts thereof, more preferably alendronate sodium, even more preferably alendronate sodium monohydrate.

As used herein the term "IDD" means impurity and degradation determination. All IDD in the present application refers to impurity and degradation determination of vitamin D or derivatives thereof, preferably cholecalciferol.

In a first aspect, the present invention provides a pharmaceutical delivery system comprising:

i) an inert core,
ii) an inner layer comprising vitamin D or a derivative thereof, preferably cholecalciferol, an emulsifier and an antioxidant, and
iii) an outer protective layer.

In a preferred embodiment, the pharmaceutical delivery system is a multiparticulate, such as a pellet, a bead, a sphere etc. Preferably the drug delivery system is in the form of a pellet.

In one embodiment, the inner layer may be applied directly to the inert core. Additionally or alternatively, the outer protective layer may be applied directly to the inner layer.

The inert core provides a substrate to which the inner layer may be applied. The inert core may be a multiparticulate, such as a granule, a pellet, a bead, a beadlet, a microcapsule, a sphere (e.g. a millisphere) etc.. Preferably, the inert core is a pellet, a bead, or a sphere. The inert core may be made up of any suitable material, or mixture of materials, such as, for example: sugars, polysaccharides, starches, cellulosic material, inorganics (e.g. glass), and polyols. Preferably, the inert core is made up of glass or sugars. Most preferably, inert core is a sugar sphere or a glass bead.

In particular, the inert core may be formed of microcrystalline cellulose. For example, the inert core may be microcrystalline cellulose pellets. Such microcrystalline cellulose pellets are commercially available and sold under the trade name Cellets™ e.g. Cellets™ 200-350.

Preferably, the inert core is a pellet formed of microcrystalline cellulose, glass or sugars.

The inert core may constitute between about 30 and about 90% (wt/wt) of the pharmaceutical delivery system, preferably between about 30 and about 60% (wt/wt), more preferably between about 30 and about 40% (wt/wt), even more preferably between about 31 and about 36% (wt/wt).

The drug layer is applied using an emulsion comprising vitamin D, derivatives thereof, preferably cholecalciferol, or combinations thereof, and an anti-oxidant. The emulsion may be based on any solvent system suitable for applying the inner layer. Preferably, the solvent system is an oil in water emulsion. In one embodiment, the solvent employed in the emulsion is an organic solvent/water mixture, more specifically an organic solvent in water emulsion. For example, the organic solvent may be medium chain triglycerides. Preferably, when the vitamin D or derivatives is cholecalciferol, the cholecalciferol to solvent ratio is between about 1:20 to about 1:60, more preferably about 1:30. The process for applying the first coating layer to the inert core is described further below.

In a preferred embodiment, the drug delivery system has a maximum diameter of about 600 microns, preferably about 400 to about 600 microns, more preferably about 500 microns or less. Such a maximum diameter may be determined by passing a sample through a 30 mesh sieve.

In a further preferred embodiment, the emulsifier achieves an emulsion having a drop size diameter of about 1 to about 5 microns, preferably about 1 to about 2 microns as measured by optical microscope. Further, the emulsifier is preferably chosen to provide a "dense" layer. A "dense" layer describes a layer which is layered on an inert core in such a way so as to provide an inner layer having a desired density. The desired density is achieved, for example when not less than 85% of the inert core is in a range of about 200 to about 350 microns and the amount of emulsion to be layered on the inert core is 3.5g emulsion per gram of inert core and the application of the inner layer provides coated pellets with a diameter of about 600 microns or less, preferably about 400 to about 600 microns, more preferably 500 microns or less.

The emulsifier may be a polaxamer, a polyethylene glycol ethyl ester, a propylene glycol or derivative thereof, acacia, copovidone or combination thereof. Polyvinyl alcohol, polyvinyl alcohol-polyethylene glycol graft copolymer (Kollicoat IR) and gelatine may also be used although for the present invention these are not as preferred as copovidone and acacia. For example, the emulsifier may be acacia or copovidione. The emulsifier may constitute between about 10 and about 30% (wt/wt) of the pharmaceutical delivery system, preferably between about 15 and about 25% (wt/wt), more preferably about 20% (wt/wt).

The antioxidant is employed in the inner layer in order to avoid oxidation of both the active ingredient and the organic solvent e.g. medium chain triglycerides. The antioxidant may be selected from tocopherol (e.g. alpha-tocopherol), ascorbic acid, sodium ascorbate, butylated hydroxyanisole, butylated hydroxytoluene and combination thereof. For example, the anti-oxidant may be butylated hydroxytoluene.

The anti-oxidant may constitute between about 0.1% (wt/wt) and about 2% (wt/wt) based on the weight of the pharmaceutical delivery system, preferably between about 0.4% (wt/wt) and about 1.0% (wt/wt), more preferably about 0.4% (wt/wt) or about 0.9% (wt/wt). Yet more preferably, the anti-oxidant is in constant ratio to cholecalciferol, e.g. cholecalciferol to anti-oxidant ratio is between about 1:1 to about 1:10, and most preferably about 1:4.

The inner layer may also further comprise an additional film former. The film former is capable of forming a solution/dispersion/emulsion and when dried is used as a robust layer. For example, the film former may be selected from sugars, such as lactose, maltose, isomalt, sucrose, starch, xylitol mannitol and combination thereof. In particular, the additional film former may be sucrose.

The additional film former may constitute between about 5 and about 30% (wt/wt) of the pharmaceutical delivery system, preferably between about 10 and about 25% (wt/wt), more preferably between about 10 and about 20% (wt/wt).

If the inner layer comprises an additional film former, the emulsion employed during the application of the inner layer described above further comprises a film former.

The outer protective layer may be any type of coating known in the art suitable for use as a protective layer in a pharmaceutical composition. In particular, a layer, at least one layer or more, which provides adequate protection against oxygen, moisture and light penetration is suitable for use as the protective layer employed in the invention. Preferably, the outer protective layer comprises coating excipients including polyvinyl alcohol (PVA) or hydroxypropyl methyl cellulose (HPMC). For example, the protective layer may be Opadry II 85F18378. Materials with brand name Opadry II and serial 85F are based on PVA which ensure favourable protection against oxygen penetration. Opadry II 85F18378 White has four constituents - titanium dioxide (E171), polyvinylalcohol, macrogol 3350 and talc. Nevertheless, another coating materials based on HPMC and its combinations with lactose, sucrose and other sugars and sugar alcohols may be used. The outer protective layer is preferably in an amount of about 10 to about 30% (wt/wt), more preferably about 20% (wt/wt) based on the weight of the pharmaceutical delivery system. In one embodiment, the outer protective layer is a top coat forming a layer around the outside of the pharmaceutical delivery system.

In another embodiment, the loss of active ingredient from the pharmaceutical delivery system is not more than about 4 percent, preferably about 3 percent or less after storage in a container filled with nitrogen at 40°C & 75% RH for 3 months, compared to the initial amount at time zero.

In one embodiment of the invention, the pharmaceutical delivery system further comprises a second active pharmaceutical ingredient. Preferably, the pharmaceutical delivery system of the present invention further comprises a bisphosphonate such as alendronate, risedronate, ibandronate, zolendronate or a salt thereof. More preferably, the bisphosphonate is alendronate sodium; in particular alendronate sodium monohydrate is preferred. In one embodiment, the inner layer further comprises a second active pharmaceutical active agent such as described above.

Alternatively, the drug delivery system may contain only a single active pharmaceutical ingredient, i.e. vitamin D or a derivative thereof, preferably cholecalciferol.

In a second aspect, the present invention provides a pharmaceutical composition comprising a pharmaceutical delivery system according to any embodiment of the first aspect of the present invention described herein.

In particular, the pharmaceutical composition further comprises a second active pharmaceutical ingredient. Preferably, the pharmaceutical composition of the present invention further comprises a bisphosphonate such as alendronate, risedronate, ibandronate, zolendronate or a salt thereof. More preferably, the bisphosphonate is alendronate sodium; in particular alendronate sodium monohydrate is preferred. More preferably, the pharmaceutical composition comprises a bisphosphonate as described above and a pharmaceutical delivery system comprising cholecalciferol.

The second active pharmaceutical ingredient may optionally be included as part of the drug delivery system described above, e.g. as part of the inner layer described above. Alternatively and preferably, the second active pharmaceutical ingredient may be included in a part of the pharmaceutical composition which is not the drug delivery system described above. Consequently, in one embodiment of the invention, the pharmaceutical composition comprises a drug delivery system as described above, a second active pharmaceutical ingredient, and at least one pharmaceutically acceptable excipient. Preferably, the second active pharmaceutical ingredient is a bisphosphonate such as alendronate, risedronate, ibandronate, zolendronate or a salt thereof. More preferably, the bisphosphonate is alendronate or a salt thereof, even more preferably the bisphosphonate is alendronate sodium; in particular alendronate sodium monohydrate is preferred.

In a preferred embodiment, the pharmaceutically acceptable excipient is selected from the list comprising a filler, a glidant or a combination thereof. Preferably, the filler is mannitol, microcrystalline cellulose or a combination thereof. Said glidant is preferably colloidal silicone dioxide.

In a particular preferred embodiment, the pharmaceutical composition comprises a pharmaceutical delivery system as described herein, alendronate, preferably alendronate sodium monohydrate, mannitol, microcrystalline cellulose, colloidal silicone dioxide and a lubricant. Preferably, said lubricant is magnesium stearate.

Any conventional tabletting technique may be employed to prepare the pharmaceutical composition of the present invention such as granulation (wet or dry), and direct compression. However, dry granulation and direct compression are preferred.

In a third aspect, the present invention provides a stable pharmaceutical composition comprising a drug delivery system wherein the drug delivery system comprises:

    i) an inert core;
    ii) an inner layer comprising vitamin D or a derivative thereof, preferably cholecalciferol, an emulsifier, and an anti-oxidant; and
    iii) an outer protective layer.

In a preferred embodiment, the loss of active ingredient is not more than about 5 percent, preferably about 4 percent or less after standard accelerated conditions (40˚C & 75% RH for 3 months) or intermediate test conditions (30˚C & 65% RH for 12 months), compared to the initial amount at Time Zero.

Preferably, a composition of the invention contains a level of total impurities and degradation products of about 4 percent or less, about 2 percent, preferably about 1 percent or less after 6 or 12 months of storage under intermediate test conditions of a temperature of about 30˚C and relative humidity of about 65 percent. More preferably, a composition of the invention contains level of total impurities and degradation products of about 1.5 percent, preferably 1.2 percent or less at Time Zero and/or about 4 percent, preferably 3 percent, more preferably 2 percent, yet more preferably about 1.5 percent after 3 months of storage under accelerated conditions of a temperature of about 40˚C and relative humidity of about 75 percent.

In a preferred embodiment, a composition of the invention contains a level of individual impurity of not more than about 1 percent, preferably about 0.8 percent or less, more preferably about 0.6 percent or less, most preferably about 0.5 percent or less after 3 months of storage under accelerated conditions of a temperature of about 40˚C and relative humidity of about 75 percent.

In another preferred embodiment, the pharmaceutical composition comprises a stable pharmaceutical grade formulated particles, more preferably pellets, of vitamin D or derivatives thereof, preferably cholecalciferol or derivatives thereof.

In a fourth aspect, the present invention provides a process for preparing a pharmaceutical drug delivery system comprising vitamin D or a derivative thereof, preferably cholecalciferol, wherein said process comprises:

    i) applying an inner coating layer to an inert core to provide a vitamin D or derivative thereof-coated core, wherein the inner coating layer comprises vitamin D or a derivative thereof, preferably cholecalciferol, an emulsifier and an anti-oxidant; and
ii) applying an outer protective layer to the resulting vitamin D or derivative thereof-coated core.

The inert core, emulsifier and anti-oxidant are described above. Suitable protective layers are also described above.

In the coating process of step i), the inert core may be coated with the inner coating layer in a number of ways suitable for applying a coating onto a substrate. A coating process may involve spraying a coating emulsion onto the inert core. In such a spraying process, the coating emulsion can be prepared by emulsifying vitamin D or a derivative thereof, preferably cholecalciferol, and an anti-oxidant in a suitable solvent with an emulsifier. For example, the coating emulsion can be sprayed onto the inert core using a fluid bed coating bottom spray system, such as a Wurster coating system.

The solvent may be any solvent suitable for use in such a coating process. The solvent may be water.

Preferably, the coating emulsion is an emulsion of vitamin D or a derivative thereof, preferably cholecalciferol, in water and an organic solvent in the presence of an anti-oxidant and an emulsifier. More preferably, the coating emulsion is an emulsion of vitamin D or a derivative thereof, preferably cholecalciferol, in water and medium chain triglycerides.

In a preferred embodiment, the coating process of step i) involves:

a) dissolving vitamin D or a derivative thereof, preferably cholecalciferol, and an anti-oxidant in a suitable solvent, preferably under heating, to provide a first solution;

b) dissolving an emulsifier and an additional film former in a suitable solvent to provide a second solution;

c) dispersing said first solution in said second solution using a homogenizer to provide a homogenised coating emulsion;

d) coating an inert core with said homogenised coating emulsion to provide a vitamin D or a derivative thereof-coated core.

In step a) above the suitable solvent is preferably medium chain triglycerides. In step b) above, the suitable solvent is preferably water.

In a preferred embodiment, the coating process of step ii) involves dispersing coating excipients comprising PVA or HPMC, e.g. Opadry II 85F18378, in water to provide a dispersion and coating the vitamin D or a derivative thereof-coated core with said dispersion. The dispersion may be applied in any suitable way, for example by top spray or bottom spary. Preferably, the vitamin D or a derivative thereof-coated core is coated with the dispersion using a Glatt fluid bed coating bottom spray system, such as a Wuster coating system).

In a fifth aspect, the present invention provides a pharmaceutical delivery system comprising an inert core, an inner layer, and an outer protective layer wherein the inner layer comprises vitamin D or a derivative thereof, preferably cholecalciferol, an emulsifier, an anti-oxidant and optionally a bisphosphonate such as alendronate.

In a sixth aspect, the present invention provides a pharmaceutical composition comprising a pharmaceutical delivery system according the fifth aspect of the invention detailed above.

In a seventh aspect, the present invention provides a stable pharmaceutical composition comprising a drug delivery system which comprises an inert core, an inner layer comprising vitamin D or a derivative thereof, preferably cholecalciferol, and, optionally, alendronate, and an outer protective layer. Preferably, the pharmaceutical composition comprises a bisphosphonate as described above, at least one excipient, and a pharmaceutical delivery system as described herein which contains cholecalciferol as the only active pharmaceutical ingredient.

In a preferred embodiment, the at least one excipient is selected from the list comprising a filler, a glidant or a combination thereof. Preferably, the filler is mannitol, microcrystalline cellulose or combination thereof. Said glidant is preferably colloidal silicone dioxide.

For example, , the pharmaceutical composition comprises the pharmaceutical delivery system, alendronate sodium monohydrate, mannitol, microcrystalline cellulose, colloidal silicone dioxide and a lubricant. Preferably, said lubricant is magnesium stearate.

In an eighth aspect, the present invention provides a process for preparing a drug delivery system comprising vitamin D or a derivative thereof, preferably cholecalciferol and a bisphosphonate, said process comprising:

i) applying an inner coating layer to an inert core to provide a vitamin D or a derivative thereof/bisphosphonate-coated core, wherein the inner coating layer comprises vitamin D or a derivative thereof, preferably cholecalciferol, bisphosphonate, an emulsifier, and an anti-oxidant; and

ii) applying an outer protective layer to the vitamin D or a derivative thereof/bisphosphonate-coated core.

In an ninth aspect, the present invention provides a process for preparing a pharmaceutical composition comprising vitamin D or a derivative thereof, preferably cholecalciferol, and a bisphosphonate, preferably alendronate, said process comprising dry granulation of a bisphosphonate and at least one excipient (for example, by compaction or slugging, passing the slugs through a mill or an oscillating granulator) to form granules and admixing the granules with a drug delivery system described herein. The admixture may subsequently be sieved and filled into a capsule or be compressed into a tablet. As an alternative to dry granulation, a dry blend of the bisphosphonate and at least one excipient with the drug delivery system comprising vitamin D or a derivative thereof, preferably cholecalciferol, may be compressed directly into a compacted dosage form. In this aspect of the invention, said drug delivery system preferably does not contain a bisphosphonate. Instead, the bisphosphonate is contained in a different part of the pharmaceutical composition.

It has been found to be possible to provide a stable pharmaceutical composition comprising vitamin D or a derivative thereof, preferably cholecalciferol. Preferred pharmaceutical formulation is in a form of a solid dosage form, preferably capsules or tablets.

Having described the invention with reference to certain preferred embodiments, other embodiments will become apparent to one skilled in the art from consideration of the specification. The invention is further defined by reference to the following examples. It will be apparent to those skilled in the art that many modifications, both to materials and methods, may be practiced without departing from the scope of the invention.

Examples

Examples 1-3

| Example | Ex. 1 | Ex.2 | Ex.3 | Ex.4 |
|---|---|---|---|---|
| Ingredients | Contains, mg/g | | | |
| Cholecalciferol | 1.25 | 1.25 | 2.50 | 1.25 |
| Medium Chain Triglycerides | 38.00 | 38.00 | 76.00 | 38.00 |
| Sucrose | 200.00 | 200.00 | 200.00 | 100.00 |
| Copovidone | ------- | 200.00 | 200.00 | 100.00 |
| Acacia | 200.00 | ------- | ------- | ------- |
| Microcrystalline Cellulose (Cellets 200-350) | 356.00 | 356.00 | 312.00 | 556.00 |
| Butylated Hydroxytoluene | 4.75 | 4.75 | 9.50 | 4.75 |
| Opadry II 85F18378 White | 200.00 | 200.00 | 200.00 | 200.00 |

Example 1:

1. 7.41 g of Butylated Hydroxytoluene and 1.95 g of Cholecalciferol were dissolved under $N_2$ in 59.28 g of hot (about 40 ˚ C) Medium chain triglycerides, using closed vessel.

2. 312 g of Acacia and 312 g of Sucrose were dissolved under $N_2$ in 1248 g of Purified water, using closed vessel.

3. Solution from step 1 was dispersed under $N_2$ in the solution from step 2 using a homogenizer.

4. 462.8 g of Microcrystalline Cellulose (Cellets 200-350) were coated with 1617 g of the emulsion from step 3 using Glatt fluid bed coating bottom spray system (Wurster coating).

5. 292.5 g of Opadry II 85F18378 White were dispersed in 900 g of Purified water.

6. Drug coated pellets from step 4 were coated with 1125 g of the dispersion from step 5, using Glatt fluid bed coating bottom spray system (Wurster coating).

Example 2:

1. 7.41 g of Butylated Hydroxytoluene and 1.95 g of Cholecalciferol were dissolved under $N_2$ in 59.28 g of hot (about 40 ˚ C) Medium chain triglycerides, using closed vessel.
2. 312 g of Copovidone and 312 g of Sucrose were dissolved under $N_2$ in 1248 g of Purified water, using closed vessel.
3. Solution from step 1 was dispersed under $N_2$ in the solution from step 2 using a homogenizer.
4. 462.8 g of Microcrystalline Cellulose (Cellets 200-350) were coated with 1617 g of the emulsion from step 3 using Glatt fluid bed coating bottom spray system (Wurster coating).
5. 292.5 g of Opadry II 85F18378 White were dispersed in 900 g of Purified water.
6. Drug coated pellets from step 4 were coated with 1125 g of the dispersion from step 5, using Glatt fluid bed coating bottom spray system (Wurster coating).

Example 3:

1. 783.75 g of Butylated Hydroxytoluene and 206.25 g of Cholecalciferol were dissolved under $N_2$ in 6270 g of hot (about 40 ˚ C) Medium chain triglycerides, using closed vessel.
2. 16.5 kg of Copovidone and 16.5 g of Sucrose were dissolved under $N_2$ in 50 kg of Purified water, using closed vessel.
3. Solution from step 1 was dispersed under $N_2$ in the solution from step 2 using a homogenizer.
4. 23.4 kg of Microcrystalline Cellulose (Cellets 200-350) were coated with 82.9 kg of the emulsion from step 3 using Glatt fluid bed coating bottom spray system (Wurster coating).
5. 18 kg of Opadry II 85F18378 White were dispersed in 54 kg of Purified water.
6. Drug coated pellets from step 4 were coated with 52.6 kg of the dispersion from step 5, using Glatt fluid bed coating bottom spray system (Wurster coating).

Example 4:

1. 326.563 g of Butylated Hydroxytoluene and 85.938 g of Cholecalciferol were dissolved under $N_2$ in 2613 g of hot (about 40˚C) Medium chain triglycerides, using closed vessel.
2. 6.875 kg of Copovidone and 6.875 kg of Sucrose were dissolved under $N_2$ in 20.8 kg of Purified water, using closed vessel.
3. Solution from step 1 was dispersed under $N_2$ in the solution from step 2 using a homogenizer.
4. 34.75 kg of Microcrystalline Cellulose (Cellets 200-350) were coated with 34.16 kg of the emulsion from step 3 using Glatt fluid bed coating bottom spray system (Wurster coating).
5. 15 kg of Opadry II 85F18378 White were dispersed in 45 kg of Purified water.
6. Drug coated pellets from step 4 were coated with 50.0 kg of the dispersion from step 5, using Glatt fluid bed coating bottom spray system (Wurster coating).

Examples 5-7

The following examples illustrate formulations comprising cholecalciferol in combination with alendronate sodium monohydrate.

**Alendronate 70 mg & Cholecalciferol 70 $\mu$g /140 $\mu$g tablets**

| Example Ingredients | Ex. 6 | Ex.6 | Ex.7 |
|---|---|---|---|
| | Contains, mg/tablet | | |
| Cholecalciferol Pellets from Ex.1 | 56.0 | ------- | ------- |
| Cholecalciferol Pellets from Ex.2 | ------- | 56.0 | ------- |
| Cholecalciferol Pellets from Ex.3 | ------- | ------- | 56.0 |
| Alendronate Sodium Monohydrate | 81.2 | 81.2 | 81.2 |
| Mannitol | 126.3 | 135.3 | 144.3 |
| Microcrystalline Cellulose | 50.0 | 40.0 | 30.0 |
| Colloidal Silicone Dioxide | 3.0 | 3.0 | 4.0 |
| Magnesium Stearate | 3.5 | 4.5 | 4.5 |
| Theoretical Tablet Weight | 320.0 | 320.0 | 320.0 |

Example 5 (Alendronate 70 mg & Cholecalciferol 70 $\mu$g tablets):

1. 243.6 g of Alendronate Sodium Monohydrate, 150 g of Microcrystalline Cellulose and 90 g of Mannitol were mixed together, passed through a 20 Mesh screen and mixed in a Y-blender for 15 minutes.
2. 4.5 g of Magnesium Stearate was passed through a 50 Mesh screen, added to the blend from step 1, and mixed in a Y-blender for 5 minutes.
3. The blend was pressed into slugs using a rotor tablet press with round flat punches.
4. 470.8 g of the slugs were milled through 1 mm screen together with 8.68 g of Colloidal Silicone Dioxide.
5. 155.37 g of Cholecalciferol Pellets from Ex. 1 and 267.18 g of Mannitol were mixed together, passed through an 18 Mesh screen and mixed with 459.73 g of the material from step 4 in a Y-blender for 15 minutes.
6. 5.55 g of Magnesium Stearate was passed through a 50 Mesh screen, added to the blend from step 5, and mixed in a Y-blender for 5 minutes.
7. The final blend from step 6 was pressed into tablets using a rotor tablet press with capsule shaped punches.

Example 6 (Alendronate 70 mg & Cholecalciferol 70 $\mu$g tablets):

1. 324.8 g of Alendronate Sodium Monohydrate, 160 g of Microcrystalline Cellulose and 156 g of Mannitol were mixed together, passed through a 25 Mesh screen and mixed in a Y-blender for 15 minutes.
2. 10 g of Magnesium Stearate was passed through a 50 Mesh screen, added to the blend from step 1, and mixed in a Y-blender for 5 minutes.
3. The blend was pressed into slugs using a rotor tablet press with round flat punches.
4. 561.48 g of the slugs were milled through 1 mm screen together with 10.91 g of Colloidal Silicone Dioxide.
5. 198.8 g of Cholecalciferol Pellets from Ex.2 and 341.87 g of Mannitol were mixed together, passed through an 18 Mesh screen and mixed with 588.25 g of the material from step 4 in a Y-blender for 15 minutes.
6. 7.1 g of Magnesium Stearate was passed through a 50 Mesh screen, added to the blend from step 5, and mixed in a Y-blender for 5 minutes.
7. The final blend from step 6 was pressed into tablets using a rotor tablet press with capsule shaped punches.

Example 7 (Alendronate 70 mg & Cholecalciferol 140 $\mu$g tablets):

1. 8120 g of Alendronate Sodium Monohydrate and 200 g of Colloidal Silicone Dioxide were mixed in a Y-blender for 5 minutes and passed through 0.8mm screen using Quadro Comil milling machine.
2. Blend from step 1, 3000 g of Microcrystalline Cellulose and 13000 g of Mannitol were mixed in a Y-blender for 15 minutes.

3. 250 g of Magnesium Stearate was passed through a 50 Mesh screen, added to the blend from step 2, and mixed in a Y-blender for 5 minutes.

4. The blend was pressed into slugs using a rotor tablet press with round flat punches.

5. The slugs were milled through 0.8 mm screen.

6. Milled slugs from step 5, 539 g of Cholecalciferol Pellets from Ex.3, 200 g of Colloidal Silicone Dioxide and 1491 g of Mannitol were mixed in a Y-blender for 5 minutes, passed through a 20 Mesh screen, returned to Y-blender and mixed for 15 minutes.

7. 200 g of Magnesium Stearate was passed through a 50 Mesh screen, added to the blend from step 6, and mixed in a Y-blender for 5 minutes.

8. The final blend from step 7 was pressed into tablets using a rotor tablet press with capsule shaped punches.

Example 8 Cholecalciferol pellets and Alendronate 70 mg & Cholecalciferol 70μg /140μg tablets - Stability test results of Cholecalciferol:

| Description, test conditions: 40˚ C, 75% RH | Test Interval | %Assay of Cholecalciferol |
|---|---|---|
| Cholecalciferol pellets 2.5 mg/g, Ex.3 Plasdone S-630 as an emulsifier Packaging - Aluminium bag filled with nitrogen | Time 0 | 101.1 |
| | 1 Month | 101.7 |
| | 2 Month | 99.3 |
| | 3 Month | 99.0 |
| | | |
| Alendronate 70 mg & Cholecalciferol 70 μg tablets, Ex. 5 Packaging - blister Alu-Alu | Time 0 | 104.7 |
| | 1 Month | 103.7 |
| | 2 Month | 106.2 |
| | 3 Month | 104.2 |
| | 6 Month | 97.5 |
| Alendronate 70 mg & Cholecalciferol 70 μg tablets Ex. 6 Packaging - blister Alu-Alu | Time 0 | 107 |
| | 1 Month | 114.6 |
| | 2 Month | 115.2 |
| | 3 Month | 112.8 |
| | 6 Month | 113.7 |
| | | |
| Aendronate 70 mg & Cholecalciferol 140 μg tablets Ex.7 Packaging - blister Alu-Alu | Time 0 | 100.1 |
| | 1 Month | 101.0 |
| | 2 Month | 104.4 |
| | 3 Month | 97.7 |

| Description, test conditions: 30˚ C, 65% RH | Test Interval | %Assay of Cholecalciferol | % Total IDD of Cholecalciferol |
|---|---|---|---|
| Alendronate 70 mg & Cholecalciferol 140 μg tablets from Ex.7. Packaging - blister Alu-Alu | Time 0 | 100.4 | 1.2 |
| | 6 Month | 99.2 | 0.91 |
| | 9 Month | 98.9 | 1.01 |
| | 12 Month | 96.7 | 1.01 |

| Description, test conditions: 40° C, 75% RH | Test Interval | %Assay of Cholecalciferol | %Total IDD of Cholecalciferol |
|---|---|---|---|
| Alendronate 70 mg & Cholecalciferol 140 μg tablets from Ex.7. Packaging - blister Alu-Alu | Time 0 | 100.1 | 1.2 |
| | 1 Month | 101.0 | 0.89 |
| | 2 Month | 104.4 | 1.13 |
| | 3 Month | 97.7 | 1.45 |

| Description, test conditions: 40° C, 75% RH | Test Interval | %Greatest unknown impurity of Cholecalciferol |
|---|---|---|
| Alendronate 70 mg & Cholecalciferol 140 μg tablets from Ex.7. Packaging - blister Alu-Alu | Time 0 | 0.4 |
| | 1 Month | 0.44 |
| | 2 Month | 0.43 |
| | 3 Month | 0.39 |
| | 6 Month | 0.39 |

| Description, test conditions: 40° C, 75% RH | Test Interval | %Assay of Cholecalciferol |
|---|---|---|
| Alendronate 70 mg & Cholecalciferol 140 μg tablets when commercial product (Vitamin D3 100 SD/S from DSM Nutritional Products, Inc.) comprises Cholecalciferol was used. Packaging - blister Alu-Alu | Time 0 | 100.3 |
| | 1 Month | 97.5 |
| | 2 Month | 96.4 |
| | 3 Month | 92.0 |
| Description, test conditions: 25° C, 65% RH | Test Interval | %Assay of Cholecalciferol |
| Alendronate 70 mg & Cholecalciferol 140 μg tablets when commercial product (Vitamin D3 100 SD/S from DSM Nutritional Products, Inc.) comprises Cholecalciferol was used. Packaging - blister Alu-Alu | Time 0 | 100.3 |
| | 3 Month | 98.3 |
| | 6 Month | 96.6 |
| | 9 Month | 95.5 |
| | 12 Month | 93.5 |

Stability was determined by an HPLC method with the following parameters:

| | |
|---|---|
| Column & Packing: | Ace C18, 3μ, 15cm × 4.6mm |
| Pre column: | Betabasic C18, 2cm × 4mm |
| Column Temperature: | 27°C |
| Detector: | UV at 265 nm and 220nm, 10 mm flow cell path length. The method should be applied only on HPLC system with dual wavelength detector. |
| Injection Volume: | 50 μL |
| Diluent: | IPA : 0.5% sodium dodecyl sulfate (SDS) solution (60:40v/v). |

**Injector Wash Solution:**      Methanol

**Autosampler Temperature:**   5˚C ±2˚C

**Mobile Phase:**      Eluent A: Acetonitrile
                      Eluent B: Purified water

**Gradient Time Program:**

| Time (min) | Flow (ml/min) | Eluent A | Eluent B |
|---|---|---|---|
| 0 | 1.0 | 50 | 50 |
| 5 | 1.0 | 50 | 50 |
| 8 | 1.0 | 100 | 0 |
| 49 | 1.0 | 100 | 0 |
| 50 | 1.0 | 50 | 50 |
| 55 | 1.0 | 50 | 50 |

Assay and impurity and degradation product were calculated as follows:
For assay:

$$\frac{\text{Total Smp. peak area}}{\text{Avg. Std. peak area}} \times \frac{\text{Std. conc.}_{(mg/ml)}{}^{*} \times \text{Vsmp}_{(ml)}}{L} \times \frac{\text{Avg. tab. weight}_{(mg)}}{\text{Smp. Wt}_{(mg)}} \times 100 =$$

$$= \% \text{ Assay of Cholecalciferol of the labeled amount}$$

$$Total\ Peak\ Area = \Pr e\ Cholecalciferol\ Peak\ Area \times 2 + Cholecalcirol\ Peak\ Area$$

For impurities and degradation products:

$$\frac{\text{Imp. peak area}}{\text{Avg. IDD Std. peak area}^{**}} \times \frac{\text{Std. conc.}_{(mg/ml)}{}^{*} \times \text{Vsmp}_{(ml)}}{L \times \text{Smp. Wt}_{(mg)} \times \text{RRf}} \times 100 = \% \text{ of IDD}$$

*Take into account the % assay and the % water of the relevant standard

** Avg. IDD std. peak area - at 265nm or at 220nm in respective to the IDD at 265nm or 220nm.

Vsmp - Sample volume

L - Labeled amount: mg Cholecalciferol / mg of pellets

RRf = Relative Response factor. RRf is equal 1.0 for all identified and not identified impurities and degradation products.

Example 8: Type of emulsifier

| Emulsifier | Drops size, $\mu$m |
|---|---|
| Acacia | 2-5 |
| Polyvinyl alcohol-polyethylene glycol graft copolymer (Kollicoat IR) | 1-2 |
| PVA | 1-2 |
| Gelatin | 1-5 |
| Copovidone (Plasdone S-630) | 1-2 |

Drops size of the emulsion as a result of using the tested polymers, as described in the table above, was determined by measuring the drops, as seen in optical microscope, by using a ruler.

The best results were achieved using acacia gum and copovidone, while copovidone provided more dense, more stable emulsion and easier preparation.

All tested polymers provided oil/water emulsion. However, using some of them (e.g. Kollicoat IR) lead to less dense active ingredient layer and therefore, to bigger pellets. Using big pellets may cause to further tableting problems, so these are not as preferred. Other polymers (e.g. PVA and Gelatin) were found to be less suitable for the Wurster process because of gel formation during drying.

Further aspects and features of the present invention are set out in the following numbered clauses.

1. A pharmaceutical delivery system comprising:

i) an inert core,
ii) an inner layer comprising vitamin D or a derivative thereof, preferably cholecalciferol, an emulsifier and an anti-oxidant, and
iii) an outer protective layer.

2. The pharmaceutical delivery system of clause 1, wherein the inert core is a multiparticulate comprising: a granule, a pellet, a bead, a beadlet, a microcapsule or a sphere (e.g. a millisphere).

3. The pharmaceutical delivery system of clause 2, wherein the inert core is a pellet, a bead, or a sphere.

4. The pharmaceutical delivery system of any preceding clause, wherein the inert core is made up of any suitable material, or mixture of materials, such as: sugars, polysaccharides, starches, cellulosic material, inorganics (e.g. glass), and polyols, preferably the inert core is made up of glass or sugars.

5. The pharmaceutical delivery system of clause 1, wherein the inert core is a sugar sphere or a glass bead.

6. The pharmaceutical delivery system of any one of clauses 1 to 3, wherein the inert core is formed of microcrystalline cellulose.

7. The pharmaceutical delivery system of any one of clauses 1 to 3, wherein the inert core is a pellet formed of microcrystalline cellulose, glass or sugars.

8. The pharmaceutical delivery system of any preceding clause, wherein the inert core constitute between about 30 and about 90% (wt/wt) of the pharmaceutical delivery system, preferably between about 30 and about 60% (wt/wt).

9. The pharmaceutical delivery system of any preceding clause, wherein the inner layer is applied using an emulsion comprising vitamin D or a derivative thereof, preferably cholecalciferol, and an anti-oxidant.

10. The pharmaceutical delivery system of clause 9, wherein the emulsion is oil in water emulsion.

11. The pharmaceutical delivery system of clause 9, wherein the emulsion is based on an organic solvent/water mixture, preferably an organic solvent in water emulsion.

12. The pharmaceutical delivery system of clause 11, wherein the organic solvent is medium chain triglycerides.

13. The pharmaceutical delivery system of clause 11, wherein when the vitamin D derivative is cholecalciferol, the cholecalciferol to solvent ratio is between about 1:20 to about 1:60, more preferably about 1:30.

14. The pharmaceutical delivery system of any preceding clause, wherein the emulsifier achieves an emulsion having a drop size of about 1 to about 5 microns, preferably about 1 to about 2 microns as measured by optical microscope.

15. The pharmaceutical delivery system of any preceding clause, wherein the delivery system is in the form of a pellet and has a maximum diameter of about 600 microns or less.

16. The pharmaceutical delivery system of any preceding clause, wherein the emulsifier is selected from the list comprising polaxamer, polyethylene glycol ethyl ester, propylene glycol or derivative thereof, acacia, copovidone or combination thereof, preferably, the emulsifier is acacia or copovidone.

17. The pharmaceutical delivery system of any preceding clause, wherein the emulsifier is in an amount of between about 10 and about 30% (wt/wt) of the pharmaceutical delivery system, preferably between about 15 and about 25% (wt/wt), more preferably about 20% (wt/wt).

18. The pharmaceutical delivery system of any preceding clause, wherein the antioxidant is selected from tocopherol (e.g. alpha-tocopherol), ascorbic acid, sodium ascorbate, butylated hydroxyanisole, butylated hydroxytoluene and combination thereof.

19. The pharmaceutical delivery system of any preceding clause, wherein the antioxidant is in an amount of between about 0.1 % (wt/wt) and about 2.0% (wt/wt) based on the weight of the pharmaceutical drug delivery system, preferably between about 0.4% (wt/wt) and about 1.0% (wt/wt), more preferably about 0.4% (wt/wt) or about 0.9% (wt/wt).

20. The pharmaceutical delivery system of any preceding clause, wherein the vitamin D or derivative thereof, preferably cholecalciferol, to anti-oxidant ratio is between about 1:1 to about 1:10, and most preferably about 1:4.

21. The pharmaceutical delivery system of any preceding clause, wherein the inner layer comprises a film former.

22. The pharmaceutical delivery system of clause 21, wherein the film former is selected from sugars, such as lactose, maltose, isomalt, sucrose, starch, xylitol, mannitol and combination thereof, preferably, the film former is sucrose.

23. The pharmaceutical delivery system of clause 21 or 22, wherein the film former is in an amount of between about 10 and about 30% (wt/wt) of the pharmaceutical drug delivery system, preferably between about 15 and about 25% (wt/wt), more preferably between about 10 and about 20% (wt/wt).

24. The pharmaceutical delivery system of any preceding clause, wherein the outer protective layer provides adequate protection against oxygen, moisture and light penetration.

25. The pharmaceutical delivery system of any preceding clause, wherein the outer protective layer comprises polyvinyl alcohol or hydroxypropyl methylcellulose.

26. The pharmaceutical delivery system of any preceding clause, wherein the outer protective layer comprises hydroxypropyl methylcellulose in combination with lactose, and sucrose, preferably in combination also with other sugars and sugar alcohols.

27. The pharmaceutical delivery system of any preceding clause, wherein the outer protective layer is preferably in an amount of about 10 to about 30% (wt/wt), more preferably about 20% (wt/wt) based on the weight of the pharmaceutical delivery system.

28. The pharmaceutical delivery system of clause 25, wherein the outer protective layer comprises OPADRY II 85F18378 containing titanium dioxide (E171), polyvinylalcohol, macrogol 3350 and talc.

29. The pharmaceutical delivery system of any preceding clause, wherein the outer protective layer is a top coat forming a layer around the outside of the pharmaceutical delivery system.

30. The pharmaceutical delivery system of any preceding clause, wherein the pharmaceutical delivery system comprises a second active pharmaceutical ingredient.

31. The pharmaceutical delivery system of any preceding clause, wherein the loss of vitamin D or a derivative thereof, preferably cholecalciferol, from the pharmaceutical delivery system is not more than about 4 percent, preferably about 3 percent or less after storage in a container filled with nitrogen at 40˚C & 75% RH for 3 months, compared to the initial amount at Time Zero.

32. The pharmaceutical delivery system of any preceding clause, wherein the delivery system is in a pharmaceutical grade.

33. A pharmaceutical composition comprising the pharmaceutical delivery system according to any one of the preceding clauses.

34. The pharmaceutical composition of clause 33, wherein the composition comprises a second active pharmaceutical ingredient and at least one excipient.

35. The pharmaceutical composition of clause 34, wherein the second active pharmaceutical ingredient is a bisphosphonate.

36. The pharmaceutical composition of clause 35, wherein the bisphosphonate is selected from alendronate, risedronate, ibandronate, zolendronate and salt thereof.

37. The pharmaceutical composition of any one of clauses 34 to 36, wherein the second active pharmaceutical ingredient is alendronate.

38. The pharmaceutical composition of any one of clauses 33 to 37, wherein the pharmaceutical composition is stable.

39. The pharmaceutical composition of any one of clauses 33 to 38, wherein the loss of vitamin D or a derivative thereof, preferably cholecalciferol, is not more than about 5 percent, preferably about 4 percent or less after standard accelerated conditions (40˚C & 75% RH for 3 months) or intermediate test conditions (30˚C & 65% RH for 12 months), compared to the initial amount as was measured at Time Zero.

40. The pharmaceutical composition of any one of clauses 33 to 39, wherein the composition contains a level of total impurities and degradation products of vitamin D or a derivative thereof, preferably cholecalciferol, of about 4 percent or less, about 2 percent, preferably about 1 percent or less after 6 or 12 months of storage under intermediate test conditions of a temperature of about 30˚C and relative humidity of about 65 percent.

41. The pharmaceutical composition of any one of clauses 33 to 40, wherein the composition contains level of total impurities and degradation products of vitamin D or a derivative thereof, preferably cholecalciferol, of about 1.5 percent, preferably 1.2 percent or less at Time Zero and/or about 4 percent, preferably 3 percent, more preferably 2 percent, yet more preferably about 1.5 percent after 3 months of storage under accelerated conditions of a temperature of about 40˚C and relative humidity of about 75 percent.

42. The pharmaceutical composition of any one of clauses 33 to 41, wherein the composition of the invention contains a level of individual impurity of vitamin D or a derivative thereof, preferably cholecaciferol, of not more than about 1 percent, preferably about 0.8 percent or less, after 3 months of storage under accelerated conditions of a temperature of about 40˚C and relative humidity of about 75 percent.

43. The pharmaceutical composition of any one of clauses 33 to 42, wherein the composition comprises a stable pharmaceutical grade formulated particles, more preferably pellets, of vitamin D or a derivative thereof, preferably cholecalciferol.

44. The pharmaceutical composition of any one of clauses 34 to 43, wherein the at least one excipient is selected from the list comprising a filler, a glidant or combination thereof.

45. The pharmaceutical composition of clause 44, wherein the filler is mannitol, microcrystalline cellulose or combination thereof.

46. The pharmaceutical composition of clause 44 or clause 45, wherein the glidant is colloidal silicone dioxide.

47. The pharmaceutical composition of any one of clauses 33 to 46, wherein the composition comprises alendronate, mannitol, microcrystalline cellulose, colloidal silicone dioxide and a lubricant, preferably, said lubricant is magnesium stearate.

48. The pharmaceutical composition of any of clauses 33 to 47, wherein the pharmaceutical composition is in a form of a solid dosage form, preferably capsules or tablets.

49. A process for preparing a pharmaceutical drug delivery system of any one of clauses 1 to 32 comprising vitamin D or a derivative thereof, preferably cholecalciferol, wherein said process comprises:

   i) applying an inner coating layer to an inert core to provide a vitamin D or a derivative thereof-coated core, wherein the inner coating layer comprises vitamin D or a derivative thereof, preferably cholecalciferol, an emulsifier and an anti-oxidant; and
   ii) applying an outer protective layer to the resulting vitamin D or a derivative thereof -coated core.

50. The process of clause 49, wherein the inner layer is applied directly to the inert core.

51. The process of clause 49 or clause 50, wherein the outer protective layer is applied directly to the inner layer.

52. The process of any one of clauses 49 to 51, wherein the coating process of step i) involves spraying a coating emulsion onto the inert core.

53. The process of clause 52, wherein the coating emulsion is prepared by emulsifying vitamin D ro a derivative thereof, preferably cholecalciferol, and an antioxidant in a suitable solvent with an emulsifier.

54. The process of clause 52 or 53, wherein the coating emulsion is sprayed onto the inert core using a fluid bed coating bottom spray system, such as a Wurster coating system.

55. The process of clause 53, wherein the solvent is water.

56. The process of any one of clauses 52 to 55, wherein the coating emulsion is an emulsion of vitamin D or a derivative thereof, preferably cholecalciferol, in water and an organic solvent in the presence of an anti-oxidant and an emulsifier.

57. The process of clause 56, wherein the coating emulsion is an emulsion of vitamin D or a derivative thereof, preferably cholecalciferol, in water and medium chain triglycerides.

58. The process of any one of clauses 49 to 57, wherein the coating process of step i) involves:

   a) dissolving vitamin D or a derivative thereof, preferably cholecalciferol, and an anti-oxidant in a suitable solvent, preferably under heating, to provide a first solution;
   b) dissolving an emulsifier and an additional film former in a suitable solvent to provide a second solution;
   c) dispersing said first solution in said second solution using a homogenizer to provide a homogenised coating emulsion;
   d) coating an inert core with said homogenised coating emulsion to provide a vitamin D or a derivative thereof, preferably cholecalciferol-coated core.

59. The process of clauses 58, wherein in step a) the suitable solvent is medium chain triglycerides.

60. The process of clause 58 or clause 59, wherein in step b) the suitable solvent is water.

61. The process of any one of clauses 49 to 60, wherein the coating process of step ii) involves dispersing coating excipients comprising polyvinyl alcohol or hydroxypropyl methylcellulose in water to provide a dispersion and coating the cholecalciferol-coated core with said dispersion.

62. The process of clause 61, wherein the vitamin D or a derivative thereof, preferably cholecalciferol-coated core is coated using a Glatt fluid bed coating bottom spray system, such as a Wuster coating system).

63. A process for preparing the pharmaceutical composition of any of clauses 33 to 48, wherein said process comprising dry granulation of a bisphosphonate and at least one excipient to form granules and admixing the granules with the drug delivery system.

64. A process for preparing the pharmaceutical composition of any of clauses 33 to 48, wherein said process comprising dry blending of the bisphosphonate and at least one excipient with the drug delivery system.

**Claims**

1. A pharmaceutical delivery system comprising:

   i) an inert core,
   ii) an inner layer comprising vitamin D or a derivative thereof, preferably cholecalciferol, an emulsifier and an anti-oxidant, and
   iii) an outer protective layer.

2. The pharmaceutical delivery system of claim 1, wherein the inert core is a multiparticulate comprising: a granule, a pellet, a bead, a beadlet, a microcapsule or a sphere (e.g. a millisphere).

3. The pharmaceutical delivery system of claim 1 or claim 2, wherein the emulsifier is selected from the list comprising polaxamer, polyethylene glycol ethyl ester, propylene glycol or derivative thereof, acacia, copovidone or combination thereof, preferably, the emulsifier is acacia or copovidone.

4. The pharmaceutical delivery system of any preceding claim, wherein the vitamin D or derivative thereof, preferably cholecalciferol, to anti-oxidant ratio is between about 1:1 to about 1:10, and most preferably about 1:4.

5. The pharmaceutical delivery system of any preceding claim, wherein the inner layer is applied using an emulsion comprising vitamin D or a derivative thereof, preferably cholecalciferol, and an anti-oxidant.

6. The pharmaceutical delivery system of claim 5, wherein the emulsion is either an oil in water emulsion or an emulsion based on an organic solvent/water mixture, preferably an organic solvent in water emulsion.

7. A pharmaceutical composition comprising the pharmaceutical delivery system according to any one of the preceding claims.

8. The pharmaceutical composition of claim 7, wherein the composition further comprises a second active pharmaceutical ingredient and at least one excipient.

9. The pharmaceutical composition of claim 8, wherein the second active pharmaceutical ingredient is a bisphosphonate, preferably the bisphosphonate is selected from alendronate, risedronate, ibandronate, zolendronate and salt thereof, more preferably the bisphosphonate is alendronate.

10. The pharmaceutical composition of any one of claims 7 to 9, wherein the at least one excipient is selected from the list comprising a filler, a glidant or combination thereof.

11. The pharmaceutical composition of any one of claims 7 to 10, wherein the composition comprises alendronate sodium monohydrate, mannitol, microcrystalline cellulose, colloidal silicone dioxide and a lubricant, preferably, said lubricant is magnesium stearate.

12. The pharmaceutical composition of any of claims 7 to 11, wherein the pharmaceutical composition is in a form of a solid dosage form, preferably capsules or tablets.

**13.** A process for preparing a pharmaceutical drug delivery system of any one of claims 1 to 6 comprising vitamin D or a derivative thereof, preferably cholecalciferol, wherein said process comprises:

> i) applying an inner coating layer to an inert core to provide a vitamin D or a derivative thereof-coated core, wherein the inner coating layer comprises vitamin D or a derivative thereof, preferably cholecalciferol, an emulsifier and an anti-oxidant; and
> ii) applying an outer protective layer to the resulting vitamin D or a derivative thereof-coated core.

**14.** The process of claim 13, wherein the inner layer is applied directly to the inert core.

**15.** A process for preparing the pharmaceutical composition of any of claims 7 to 12, wherein said process comprises either

> (i) dry granulation of a bisphosphonate and at least one excipient to form granules and admixing the granules with the drug delivery system; or
> (ii) dry blending of the bisphosphonate and at least one excipient with the drug delivery system.

Figure 1: Emulsifier - Acacia gum

Figure 2: Emulsifier – Copovidone

Figure 3: Emulsifier - Acacia gum

Figure 4: Emulsifier - Copovidone

Figure 5: Emulsifier - Acacia gum

Figure 6: Emulsifier – Copovidone

**Europäisches Patentamt**
**European Patent Office**
**Office européen des brevets**

## EUROPEAN SEARCH REPORT

Application Number

EP 09 18 0698

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/28564 A1 (YUYU IND CO LTD [KR]; KANG SUNG AN [KR]; LEE KYUNG HEE [KR]; KWAK CHUN) 26 April 2001 (2001-04-26) * claims 1,2,6 * ----- | 1-15 | INV. A61K9/20 A61K9/50 A61K31/59 A61K31/663 |
| A | WO 98/29105 A2 (BONE CARE INT INC [US]) 9 July 1998 (1998-07-09) * examples 10, 11 * ----- | 1-15 | |
| A | US 2 811 483 A (ATERNO JOSEPH P ET AL) 29 October 1957 (1957-10-29) * examples 1, 2 * * claim 1 * ----- | 1-15 | |
| A | WO 02/24165 A2 (NYCOMED PHARMA AS [NO]; COCKBAIN JULIAN [GB]; SCHLYTER JIMMY HIRSCHSPR) 28 March 2002 (2002-03-28) * page 8, line 5 - line 17 * * example 1 * ----- | 1-15 | |
| A | EP 0 820 703 A1 (VALPHARMA SA [SM]) 28 January 1998 (1998-01-28) * examples 3, 4 * ----- | 1-15 | TECHNICAL FIELDS SEARCHED (IPC) A61K |

The present search report has been drawn up for all claims

| Place of search | Date of completion of the search | Examiner |
|---|---|---|
| Munich | 6 May 2010 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 09 18 0698

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2010

| Patent document cited in search report | | Publication date | Patent family member(s) | | Publication date |
|---|---|---|---|---|---|
| WO 0128564 | A1 | 26-04-2001 | AT | 417615 T | 15-01-2009 |
| | | | AU | 1059601 A | 30-04-2001 |
| | | | CN | 1420777 A | 28-05-2003 |
| | | | EP | 1229917 A1 | 14-08-2002 |
| | | | ES | 2316392 T3 | 16-04-2009 |
| | | | JP | 2003514776 T | 22-04-2003 |
| | | | KR | 20010037885 A | 15-05-2001 |
| | | | US | 6835722 B1 | 28-12-2004 |
| WO 9829105 | A2 | 09-07-1998 | AT | 340581 T | 15-10-2006 |
| | | | AU | 724153 B2 | 14-09-2000 |
| | | | AU | 7888398 A | 31-07-1998 |
| | | | BR | 9715022 A | 18-09-2001 |
| | | | CA | 2276465 A1 | 09-07-1998 |
| | | | CN | 1251527 A | 26-04-2000 |
| | | | EP | 0951286 A2 | 27-10-1999 |
| | | | HU | 0003526 A2 | 28-03-2001 |
| | | | JP | 2001512418 T | 21-08-2001 |
| | | | MX | PA99006988 A | 02-07-2002 |
| | | | NZ | 336511 A | 31-08-2001 |
| | | | PL | 334348 A1 | 28-02-2000 |
| US 2811483 | A | 29-10-1957 | NONE | | |
| WO 0224165 | A2 | 28-03-2002 | AT | 380541 T | 15-12-2007 |
| | | | AU | 8791801 A | 02-04-2002 |
| | | | BG | 107738 A | 30-01-2004 |
| | | | CN | 1461210 A | 10-12-2003 |
| | | | CZ | 20030808 A3 | 13-08-2003 |
| | | | DK | 1320356 T3 | 28-01-2008 |
| | | | EE | 200300109 A | 15-04-2005 |
| | | | EP | 1320356 A2 | 25-06-2003 |
| | | | HU | 0301250 A2 | 28-11-2003 |
| | | | NO | 20031249 A | 20-05-2003 |
| | | | PL | 365047 A1 | 27-12-2004 |
| | | | PT | 1320356 E | 03-01-2008 |
| | | | SK | 3232003 A3 | 03-02-2004 |
| | | | US | 2004043043 A1 | 04-03-2004 |
| EP 0820703 | A1 | 28-01-1998 | IT | MI961525 A1 | 22-01-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03059358 A

- US 4997824 A

**Non-patent literature cited in the description**

- Bisphosphonates: An Overview with Special Reference to Alendronate. Ann. Clin.-4, Biochem. (2001). 2001, vol. 38, 608-623